(19) Europäisches Patentamt / European Patent Office / Office européen des brevets

(11) **EP 3 254 712 B1**

(12) **EUROPEAN PATENT SPECIFICATION**

(45) Date of publication and mention of the grant of the patent:
**23.10.2019 Bulletin 2019/43**

(21) Application number: **16746732.3**

(22) Date of filing: **05.02.2016**

(51) Int Cl.:
*A61M 1/14* (2006.01)   *A61M 1/16* (2006.01)

(86) International application number:
**PCT/JP2016/053552**

(87) International publication number:
**WO 2016/125902 (11.08.2016 Gazette 2016/32)**

(54) **TREATMENT SOLUTION PREPARATION DEVICE AND BLOOD PROCESSING SYSTEM**

VORRICHTUNG ZUR ZUBEREITUNG EINER BEHANDLUNGSLÖSUNG UND BLUTVERARBEITUNGSSYSTEM

DISPOSITIF DE PRÉPARATION D'UNE SOLUTION DE TRAITEMENT ET SYSTÈME DE TRAITEMENT DU SANG

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **06.02.2015 JP 2015022492**

(43) Date of publication of application:
**13.12.2017 Bulletin 2017/50**

(60) Divisional application:
**19156717.1 / 3 498 315**
**19158509.0 / 3 524 287**

(73) Proprietor: **Asahi Kasei Medical Co., Ltd.**
**Tokyo 100-0006 (JP)**

(72) Inventors:
• UMIMOTO, Takashi
  Tokyo 101-8101 (JP)
• TSUCHIYA, Mizuho
  Tokyo 101-8101 (JP)

(74) Representative: **dompatent von Kreisler Selting Werner -**
**Partnerschaft von Patent- und Rechtsanwälten mbB**
**Deichmannhaus am Dom**
**Bahnhofsvorplatz 1**
**50667 Köln (DE)**

(56) References cited:
WO-A1-94/11093          JP-A- H07 275 354
JP-A- H08 504 116       JP-A- 2013 081 662
JP-A- 2014 529 409      US-A1- 2007 255 527

## Description

### Technical Field

The present invention relates to a therapeutic fluid preparation device and a blood processing system.

### Background Art

Continuous blood treatment that purifies blood continuously by extracorporeal circulation is one of the therapies applicable to patients having acute renal failure and other severely ill patients. This type of treatment is typically performed by a blood processing device, and such blood processing device includes a blood processing circuit for supplying the blood of a patient to a blood purifier and then returning the purified blood to the patient, as well as a therapeutic fluid supply circuit for supplying therapeutic fluid which is to be used in the processing circuit. Examples of the therapeutic fluid include replacement fluid which is supplied to the blood within the blood processing circuit so as to maintain the body fluid balance in the patient, and dialysate which is supplied to the blood purifier so as to remove waste products from the blood of the patient.

In the continuous blood treatment, therapeutic fluid, such as dialysate and replacement fluid, is usually supplied from a bag which contains the therapeutic fluid that has already been prepared. In order to continuously process the blood in the blood processing device for a long time, healthcare workers have to replace the bag several times during the treatment, which is a burden to such healthcare workers. Furthermore, since a bag having therapeutic fluid contained therein is expensive, the use of such bag is also a considerable burden in terms of cost. Such burdens can be eliminated if it is possible to prepare therapeutic fluid, such as dialysate and replacement fluid, to be used for the blood processing in real time during the treatment and to replenish the blood processing device with the prepared therapeutic fluid. Under these circumstances, a developed technique connects a dialysate preparation device to a blood processing device so that dialysate prepared in the dialysate preparation device is supplied to a blood purification device (see Patent Document 1). WO94/11093 discloses a method and an apparatus for kidney dialysis. The apparatus comprises conductivity probes and thermistors that are calibrated using solutions with known conductivity and temperature. US2007/0255527 discloses disposable, pre-calibrated conductivity sensors for use in bio-processing applications. The sensors are calibrated using a solution with a known concentration and temperature.

Citation List

Patent Document

Patent Document 1: Japanese Patent No. 4458346

### Summary

Technical Problem

The above-mentioned dialysate preparation device needs to prepare dialysate at a desired concentration by way of mixing a plurality of stock solutions. Accordingly, the dialysate preparation device has to be equipped with a concentration measuring device for measuring the concentration of a solution mixture. For such concentration measuring device, a concentration measuring device including an electric conductivity sensor and a temperature sensor is used so that the concentration of a solution mixture can be measured accurately through the measurement of electric conductivity and temperature.

Electric conductivity sensors and temperature sensors as described above have their own margin of error, and it is accordingly necessary to calibrate the sensors before use in order to measure the concentration of a solution mixture with a high degree of accuracy.

A typical example of such calibration of a sensor may include separately attaching a calibrated, standard sensor to the circuit in the preparation stage for treatment so as to measure concentration with the standard sensor, and calibrating the sensor value so that the value is consistent with the result of the concentration measurement in the preparation stage.

However, attaching the standard sensor to the circuit and other relevant processes will be a burden to healthcare workers. Furthermore, attaching and detaching the standard sensor to/from the circuit is undesirable from the perspective of hygiene. Performing the calibration operation during the process of manufacturing the sensor is one option, but this means an increase in the number of steps in the manufacturing process. Moreover, in order to perform the calibration operation in the manufacturing process of the sensor, it becomes necessary to add a further function (means) for storing calibration information, and such addition will consequently hinder size reduction.

Moreover, in a disposable system as described in Patent Document 1, a therapeutic fluid mixing circuit, including sensors provided therein, is disposable and therefore needs calibration each time. An increase in the cost of a sensor directly affects the cost of each treatment.

The present invention has been made in view of the above circumstances. An object of the present invention is to improve the measurement accuracy of a concentration measuring device of a therapeutic fluid preparation device, without increasing the burden to healthcare workers in a preparation stage for blood treatment, such as continuous blood treatment, and also without the need to add further functions to a sensor.

Solution to Problem

In order to achieve the above object, the inventors of the present invention conducted intensive studies and, as a result, the inventors have found that the measurement accuracy of a concentration measuring device can be improved by supplying an electrolyte having a known concentration through a mixing circuit separately from a solution mixture, measuring the electric conductivity and temperature of the electrolyte with an electric conductivity sensor and a temperature sensor, and using the results of the measurement, and have accordingly completed the present invention which is defined in independent claims 1 and 4, with preferred embodiments specified in the dependent claims. That is, the present invention provides the following (1):

(1) A therapeutic fluid preparation device for preparing therapeutic fluid having a predetermined concentration by mixing a plurality of stock solutions, the device comprising: a mixing circuit configured to mix the plurality of stock solutions; a concentration measuring device having an electric conductivity sensor configured to measure the electric conductivity of a solution mixture in the mixing circuit and a temperature sensor configured to measure the temperature of the solution mixture, the concentration measuring device being configured to measure the concentration of the solution mixture in accordance with the measured electric conductivity by the electric conductivity sensor and the measured temperature by the temperature sensor; electrolyte supply means configured to supply an electrolyte whose temperature and electric conductivity is known through the mixing circuit; and calibration means configured to direct the electrolyte supply means to supply the electrolyte whose temperature and electric conductivity is known through the mixing circuit, that is configured to measure the electric conductivity of the supplied electrolyte using the electric conductivity sensor, and that is configured to calibrate the temperature sensor based on the difference between the measured electric conductivity and the known electric conductivity of the electrolyte by referring to a correlation between temperature and electric conductivity.

Although not forming part of the invention, the present disclosure also provides the following (2) to (4):

(2) A therapeutic fluid preparation device for preparing therapeutic fluid having a predetermined concentration by mixing a plurality of stock solutions, the device comprising: a mixing circuit in which the plurality of stock solutions are mixed; a concentration measuring device having an electric conductivity sensor that measures an electric conductivity of a solution mixture in the mixing circuit and a temperature sensor that measures a temperature of the solution mixture, the concentration measuring device measuring a concentration of the solution mixture in accordance with the measured electric conductivity by the electric conductivity sensor and the measured temperature by the temperature sensor; electrolyte supply means that supplies an electrolyte having a known concentration through the mixing circuit; and calibration means that directs the electrolyte supply means to supply the electrolyte having the known concentration through the mixing circuit, that measures a temperature of the supplied electrolyte using the temperature sensor, and that calibrates the electric conductivity sensor based on the difference between the measured temperature and a known temperature of the electrolyte by referring to a correlation between temperature and electric conductivity.

(3) A therapeutic fluid preparation device for preparing therapeutic fluid having a predetermined concentration by mixing a plurality of stock solutions, the device comprising: a mixing circuit in which the plurality of stock solutions are mixed; a concentration measuring device having an electric conductivity sensor that measures an electric conductivity of a solution mixture in the mixing circuit and a temperature sensor that measures a temperature of the solution mixture, the concentration measuring device measuring a concentration of the solution mixture in accordance with the measured electric conductivity by the electric conductivity sensor and the measured temperature by the temperature sensor; electrolyte supply means that supplies an electrolyte having a known concentration through the mixing circuit with a temperature of the electrolyte being varied; calibration curve generating means that directs the electrolyte supply means to supply the electrolyte having the known concentration through the mixing circuit with varying temperatures, that measures an electric conductivity of the supplied electrolyte using the electric conductivity sensor, that also measures a temperature of the supplied electrolyte using the temperature sensor, and that thereby generates a calibration curve between electric conductivity and temperature; and concentration calculating means that measures the electric conductivity and the temperature of the solution mixture in the mixing circuit using the electric conductivity sensor and the temperature sensor, that determines, on the calibration curve, the electric conductivity of the electrolyte at the same temperature as the measured temperature of the solution mixture, and that calculates the concentration of the solution mixture by using the following relational expression:

$$\text{(Electrolyte electric conductivity)} / \text{(Solution mixture electric conductivity)} = \alpha * \text{(Electrolyte concentration)} / \text{(Solution mixture concentration)},$$

where $\alpha$ is a constant.

(4) The therapeutic fluid preparation device according to (3), wherein the electrolyte supply means has a heating device provided on the mixing circuit.

In preferred embodiments, the present invention also provides the following (5) to (7):

(5) The therapeutic fluid preparation device according to (1), wherein at least one of the electric conductivity sensor and the temperature sensor is disposable.

(6) The therapeutic fluid preparation device according to (1), wherein the electrolyte is a priming solution.

(7) A blood processing system for performing continuous blood treatment, comprising a therapeutic fluid preparation device according to (1).

Advantageous Effects of Invention

According to the present invention, the measurement accuracy of a concentration measuring device of a therapeutic fluid preparation device can be improved without increasing the burden to healthcare workers in a preparatory stage for blood treatment, such as continuous blood treatment, and also without the need to add further functions to a sensor.

**Brief Description of Drawings**

Fig. 1 is an illustration showing the schematic configuration of a blood processing system.

Fig. 2 is an illustration showing the schematic configuration of a therapeutic fluid preparation device according to a first embodiment.

Fig. 3 is an illustration showing the schematic configuration of a therapeutic fluid preparation device having two concentration measurement devices.

Fig. 4 is an illustration showing the schematic configuration of a therapeutic fluid preparation device having two means for supplying an electrolyte.

Fig. 5 is an illustration showing the schematic configuration of a therapeutic fluid preparation device.

Fig. 6 is a graph showing a calibration curve between electric conductivity and temperature.

Fig. 7 is an illustration showing the schematic configuration of a therapeutic fluid preparation device.

**Description of Embodiments**

Hereinafter, preferred embodiments of the present invention will be described with reference to the attached drawings. It should be noted that the below embodiments are just examples for describing the present invention and the present invention is not limited only to the described embodiments. The same elements will be given the same reference numerals and any repetitive explanation will be omitted. In the drawings, unless otherwise specified, the positional relationships, such as up-and-down and right-and-left relationships, are based on those shown in the drawings. The various dimensional ratios shown in the drawings are not limited to those in the drawings.

First embodiment

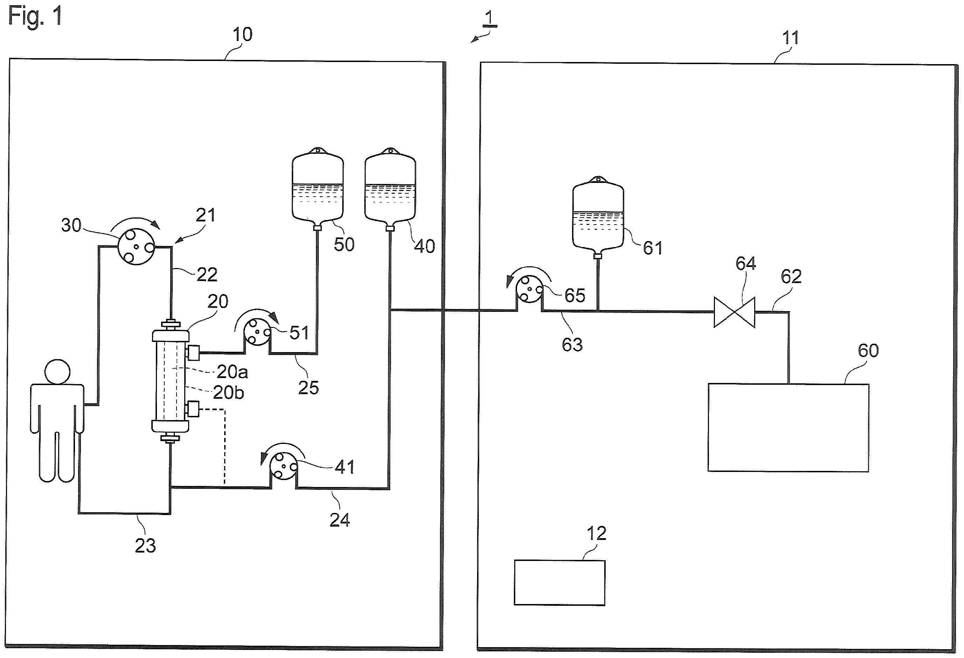

Fig. 1 is an illustration showing the schematic configuration of a blood processing system 1 equipped with a therapeutic fluid preparation device according to the present embodiment. For example, the blood processing system 1 is used for performing continuous blood treatment. The blood processing system 1 includes, for example, a blood processing device 10 for processing the blood of a patient, a therapeutic fluid supply device 11 that supplies and replenishes the blood processing device 10 with therapeutic fluid, such as dialysate and replacement fluid, and a control device 12.

The blood processing device 10 has a blood processing circuit 21 for supplying the blood of a patient into a blood purifier 20 and returning the resulting blood back to the patient. The blood processing circuit 21 includes a blood supply circuit 22 for supplying the blood of a patient into the blood purifier 20 and a blood return circuit 23 for returning the blood from the blood purifier 20 to the patient.

The blood processing device 10 also has: a therapeutic fluid supply circuit 24 which is used for at least one of the supply of replacement fluid to the blood processing circuit 21 or the blood return circuit 23, or the supply of dialysate to the blood purifier 20; and a drainage circuit 25 for discharging the waste liquid generated in the blood purifier 20.

The blood purifier 20 is, for example, a hollow fiber membrane module having a hollow fiber membrane incorporated therein, which is able to filter the blood and remove waste products, etc., in the blood by allowing the blood supplied to the primary side 20a of the hollow fiber membrane to pass through to the secondary side 20b of the hollow fiber membrane.

The blood supply circuit 22 is connected, for example, from a needle point where a needle is inserted into the patient (not shown in the drawing) to an inlet on the primary side 20a of the blood purifier 20. The blood supply circuit 22 is provided with a pump 30 so that the blood of the patient can be supplied to the blood purifier 20 at a predetermined flow rate. The pump 30 is configured to, for example, send a liquid in a tube that constitutes the circuit by squeezing the tube, which is called a tube pump.

The blood return circuit 23 is connected from an outlet on the primary side 20a of the blood purifier 20 to a needle point (not shown in the drawing).

The therapeutic fluid supply circuit 24 is connected from a reservoir container 40 to any of the blood processing circuit 21, the blood return circuit 23 or the secondary side 20b of the blood purifier 20, or to some or all of them. A selection can be made as to whether to connect the therapeutic fluid supply circuit 24 to any of the blood processing circuit 21, the blood return circuit 23 or the blood purifier 20, or whether to connect the circuit 24 to two or more of them, according to the type of blood purification treatment, such as continuous hemofiltration (CHF), continuous hemodialysis (CHD), or continuous hemodialfiltration (CHDF).

The therapeutic fluid supply circuit 24 is provided with a tube pump 41 acting as a supply pump, so that the therapeutic liquid (e.g., dialysate and replacement fluid) in the reservoir container 40 can be supplied at a predetermined flow rate.

The drainage circuit 25 is connected from the secondary side 20b of the blood purifier 20 to a drainage container 50. The drainage circuit 25 is provided with a tube pump 51 acting as a drain pump, so that the waste liquid from the blood purifier 20 can be discharged into the drainage container 50 at a predetermined flow rate.

The therapeutic fluid supply device 11 includes, for example, a therapeutic fluid preparation device 60 that prepares a therapeutic fluid, a therapeutic fluid supply circuit 62 for supplying the therapeutic fluid generated in the therapeutic fluid preparation device 60 into a reservoir container 61, and a therapeutic fluid replenishing circuit 63 for replenishing the reservoir container 40 of the blood processing device 10 with the therapeutic fluid in the reservoir container 61.

The therapeutic fluid supply circuit 62 is connected from the therapeutic fluid preparation device 60 to the reservoir container 61, while the therapeutic fluid replenishing circuit 63 is connected from the reservoir container 61 to the therapeutic fluid supply circuit 24 in the blood processing device 10. The therapeutic fluid supply circuit 62 is provided with, for example, an on-off valve 64. The therapeutic fluid replenishing circuit 63 is provided with a tube pump 65, so that the therapeutic fluid in the reservoir container 61 can be supplied to the therapeutic fluid supply circuit 24 at a predetermined flow rate.

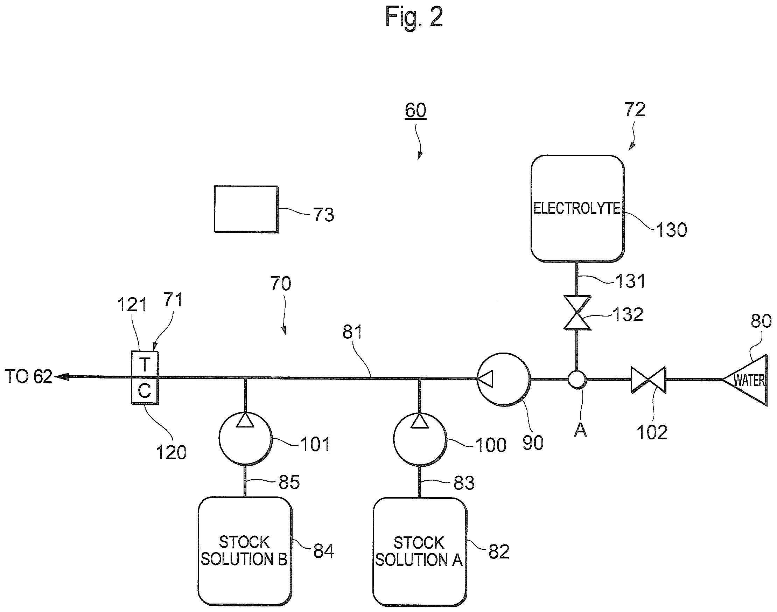

The therapeutic fluid preparation device 60 includes, for example, a mixing circuit 70 in which two or more stock solutions are mixed, a concentration measuring device 71 that measures the concentration of a solution mixture in the mixing circuit 70, an electrolyte supply means 72 that supplies an electrolyte to the mixing circuit 70, and a calibration means 73, as illustrated in Fig. 2.

The mixing circuit 70 includes, for example: a solution mixture preparation circuit 81 which is connected from a water supply 80 to the therapeutic fluid supply circuit 62; a stock solution-A supply circuit 83 which is connected from a stock solution-A supply source 82 to the solution mixture preparation circuit 81; and a stock solution-B supply circuit 85 which is connected from a stock solution-B supply source 84 to the solution mixture preparation circuit 81. For example, reverse osmosis filtrate water, ultra-filtrate water, or the like is used for water in the water supply 80. For example, a stock solution not containing sodium bicarbonate is used for stock solution-A, and a stock solution containing sodium bicarbonate is used for stock solution-B.

The solution mixture preparation circuit 81 is provided with a liquid feeding means 90, such as a pump, for sending water in the water supply 80 toward the therapeutic fluid supply circuit 62. The stock solution-A supply circuit 83 is connected to the solution mixture preparation circuit 81 at a point downstream of the liquid feeding means 90. The stock solution-A supply circuit 83 is provided with a liquid feeding means 100, such as a pump. The stock solution-B supply circuit 85 is connected to the solution mixture preparation circuit 81 at a point downstream of the connection point with the stock solution-A supply circuit 83. The stock solution-B supply circuit 85 is provided with a liquid feeding means 101, such as a pump. Furthermore, an opening/closing means 102, such as a valve, is provided in the solution mixture preparation circuit 81 at a point upstream of the liquid feeding means 90.

The concentration measuring device 71 has an electric conductivity sensor 120 that measures the electric conductivity of the solution mixture in the mixing circuit 70 and a temperature sensor 121 that measures the temperature of the solution mixture, and the concentration measuring device 71 is able to measure the concentration of the solution mixture based on the measured electric conductivity by the electric conductivity sensor 120 and the measured temperature by the temperature sensor 121. The electric conductivity sensor 120 and the temperature sensor 121 are each disposed in the solution mixture preparation circuit 81 at a point downstream of the connection point with the stock solution-B supply circuit 85. The results of measuring the electric conductivity and the temperature from the electric conductivity sensor and the temperature sensor 121 can be output to the calibration means 73.

The electrolyte supply means 72 is configured so as to supply an electrolyte having a known concentration into the solution mixture preparation circuit 81 of the mixing circuit 70 so as to allow the electrolyte to flow through the mixing circuit 70. An electrolyte having a known concentration herein means an electrolyte which has known values of electric conductivity and temperature, upon which the concentration is dependent. The electrolyte supply means 72 is comprised of, for example, an electrolyte supply source 130, an electrolyte supply circuit 131 which is connected from the electrolyte supply source 130 to the solution mixture preparation circuit 81, an opening/closing means 132, such as an electromagnetic valve or a clamping device, for opening and closing the electrolyte supply circuit 131, and the above-described liquid feeding means 90 for sending the electrolyte in the electrolyte supply source 130 to the solution mixture preparation circuit 81. A separate liquid feeding means, such as a pump, may be provided for the electrolyte supply circuit 131. The electrolyte supply circuit 131 is connected to the solution mixture preparation circuit 81, for example, at a point upstream of the liquid feeding means 90. In other words, the connection point A between the solution mixture preparation circuit 81 and the electrolyte supply circuit 131 is upstream of the liquid feeding means 90. Examples of the electrolyte used include physiological saline being a sodium ion solution.

The calibration means 73 is, for example, a computer, which calibrates, for example, the temperature sensor 121. For example, the calibration means 73 is configured to: allow an electrolyte having a known concentration to flow through the mixing circuit 70 with the aid of the electrolyte supply means 72; measure the electric conductivity of the electrolyte using the electric conductivity sensor 120; and calibrate the temperature sensor 121 based on the difference between the measured electric conductivity and a known electric conductivity of the electrolyte by referring to the correlation between temperature and electric conductivity. The details of the method for such calibration will be described later.

The mixing circuit 70, the concentration measuring device 71, and other portions of the therapeutic fluid preparation device 60, excluding the liquid feeding means 90, 100 and 101, are configured so as to be disposable members, and will be replaced each time of treatment.

The control device 12 is, for example, a computer that controls the overall operation of the blood processing system 1, and it achieves the blood processing and the replenishment of the therapeutic fluid by controlling, for example, the operation of the tube pumps 30, 41, 51 and 65 and the on-off valve 64 shown in Fig. 1, as well as the operation of the liquid feeding means 90, 100 and 101 and the opening/closing means 102 and 132 in the therapeutic fluid preparation device 60. It should be noted here that the blood processing device 10 and the therapeutic fluid supply device 11 may each have respective control computers and the control device 12 may perform overall control through wired or wireless communication. It should also be noted that the calibration means 73 may be included in the control device 12 or it may be a separate member from the control device 12.

Next, the operation of the blood processing system 1 having the above-described configuration will be described.

Calibration of concentration measuring device 71

The concentration measuring device 71 is calibrated by the calibration means 73 shown in Fig. 2, for example, in a preparation stage before starting continuous blood treatment. First, in the therapeutic fluid preparation device 60, an electrolyte having a known concentration (i.e., an electrolyte whose temperature and electric conductivity are known) in the electrolyte supply source 130 is supplied to the solution mixture preparation circuit 81 by the electrolyte supply means 72, and allowed to flow through the solution mixture preparation circuit 81. In this operation, the liquid feeding means 90 operates with the opening/closing means 132 being open and the opening/closing means 102 being closed, so that the electrolyte in the electrolyte supply source 130 flows into the solution mixture preparation circuit 81 via the electrolyte supply circuit 131. Then, the electric conductivity of the electrolyte flowing through the solution mixture preparation circuit 81 is measured by the electric conductivity sensor 120 of the concentration measuring device 71. The result of the electric conductivity measurement is output to the calibration means 73. The calibration means 73 determines the difference between the measured electric conductivity of the electrolyte measured by the electric conductivity sensor 120 and the known electric conductivity of the same electrolyte, and regards the obtained difference as being a temperature factor, so that the calibration means 73 calibrates the temperature sensor 121 based on the difference by referring to the correlation between temperature and electric conductivity.

To be more specific, the above calibration is based on the premise that electric conductivity and temperature vary with a certain correlation therebetween (electric conductivity variation $\Delta C = \beta$ x temperature variation $\Delta T$ (electric conductivity varies by $\beta$ mS/cm when temperature varies by 1°C)). The value $\beta$ of correlation is obtained in advance. Then, the difference $\Delta X$ between the measured electric conductivity measured by the electric conductivity sensor 120 and the known electric conductivity of the electrolyte is considered to be entirely due to the temperature sensor, i.e., the difference $\Delta X$ is considered to be an error of the temperature sensor 121 and is therefore converted into a calibration amount Y for the temperature sensor 121. Such calibration amount Y for the temperature sensor 121 can be obtained from the above-mentioned correlation between the temperature and electric conductivity and the difference $\Delta X$. More specifically, since the temperature varies by 1/$\beta$°C when the electric conductivity varies by 1 mS/cm, the calibration amount Y for the temperature sensor 121 can be determined by the following expression (1):

$$Y = \Delta X \times 1/\beta \dots\dots\dots (1).$$

For example, in the case where a 150 mmol/L sodium ion solution is used as an electrolyte and the electric conductivity thereof varies by 2 mS/cm when the temperature varies by 1°C (i.e., $\beta$ = 2), and if the known electric conductivity is 15 mS/cm at 25 °C and the measured electric conductivity is 14 mS/cm, the difference $\Delta X$ between the known electric conductivity and the measured electric conductivity is 1 mS/cm and the calibration amount Y for the temperature sensor 121 is calculated as: 1 x 1/2 = 0.5°C according to the above expression (1). Accordingly, the temperature sensor 121 is calibrated by the obtained calibration amount Y (0.5°C). As a result, the concentration measuring device 71, which measures concentration by way of the electric conductivity sensor 120 and the temperature sensor 121, can be calibrated. It should be noted here that the above calibration of the concentration measuring device 71 may be performed at the same time with the process of cleaning the therapeutic fluid supply circuit 62 and the mixing circuit 70, namely, a priming process. In that case, the electrolyte also serves as a priming solution.

Blood purification

The treatment of the blood starts upon completion of the preparation therefor. For example, continuous blood purification is performed for the patient in the blood processing device 10 shown in Fig. 1. In the case of performing, for example, continuous hemofiltration (CHF), the blood of the patient is supplied to the blood purifier 20 through the blood supply circuit 22 by means of the tube pump 30. When waste products, etc., are removed from the blood through the membrane in the blood purifier 20, the waste products, etc., are discharged into the drainage container 50 via the drainage circuit 25 by means of the tube pump 51. Further, the replacement fluid in the reservoir container 40 is supplied to the blood processing circuit 21 and/or the blood return circuit 23 via the therapeutic fluid supply circuit 24 by means of the tube pump 41. The blood that has been purified by the blood purifier 20 is then returned to the patient through the blood return circuit 23. In the case of performing continuous hemodialysis (CHD), the dialysate in the reservoir container 40 is supplied to the secondary side 20b of the membrane in the blood purifier 20 by means of the tube pump 41. In the case of performing continuous hemodiafiltration (CHDF), the replacement fluid is supplied to the blood processing circuit 21 and/or the blood return circuit 23 by means of the tube pump 41 and the dialysate is also supplied to the blood purifier 20 with the tube pump 41.

While the blood purification is being performed in the blood processing device 10, the therapeutic fluid supply device 11 supplies and replenishes the reservoir container 40 of the blood processing device 10, as appropriate, with the therapeutic fluid in the reservoir container 61. In this operation, the therapeutic fluid is supplied to the therapeutic fluid supply circuit 24 of the blood processing device 10 via the therapeutic fluid replenishing circuit 63 by means of the tube pump 65. The therapeutic fluid supplied to the therapeutic fluid supply circuit 24 is mainly supplied and stored in the reservoir container 40, and, in some cases, a portion of the therapeutic fluid is directly supplied to the blood return circuit 23 or the blood purifier 20.

The therapeutic fluid is prepared in the therapeutic fluid preparation device 60 while the blood treatment is stopped, and the prepared therapeutic fluid is stored in the reservoir container 61 in advance. When the amount of the therapeutic fluid in the reservoir container 61 decreases, the reservoir container 61 is replenished as appropriate. In this operation, the liquid feeding means 90, 100 and 101 operate so that stock solutions A and B that are flowing at predetermined flow rates are mixed with water that is flowing at a predetermined flow rate in the solution mixture preparation circuit 81, thereby preparing a therapeutic fluid having a desired concentration. The concentration measuring device 71 measures the concentration of the prepared therapeutic fluid and uses the measurement results to regulate the flow rates of the liquid feeding means 90, 100 and 101, so as to accordingly enable feedback control of the concentration of the therapeutic fluid.

According to the present embodiment: the electrolyte supply means 72 supplies an electrolyte having a known concentration to the mixing circuit 70 and allows it to flow though the mixing circuit 70; the electric conductivity sensor 120 measures the electric conductivity of such electrolyte having the known concentration; and the temperature sensor 121 is calibrated based on the difference between the measured electric conductivity and a known electric conductivity in accordance with the correlation between the temperature and electric conductivity. As a result, the present embodiment enables the calibration of the concentration measuring device 71 in the therapeutic fluid preparation device 60 without increasing the burden to healthcare workers during the preparation stage for treatment and also without the need to add further functions to the sensors, thereby improving the measurement accuracy of the concentration measuring device 71.

Furthermore, the concentration measuring device 71 is a disposable member. Such concentration measuring device 71 requires no maintenance operation, but it is replaced and therefore requires calibration each time of treatment. According to the present embodiment, however, the calibration of the concentration measuring device 71, which is performed in each treatment, can be easily performed, so that the burden to healthcare workers will be significantly lessened.

## Second embodiment, not forming part of the invention

The above-described first embodiment is configured to improve the measurement accuracy of the concentration measuring device 71 through the calibration of the temperature sensor 121, but the measurement accuracy of the concentration measuring device 71 may be improved by calibrating the electric conductivity sensor 120.

In that case, the calibration means 73: allows an electrolyte having a known concentration to flow through the mixing circuit 70 with the aid of the electrolyte supply means 72; measures the temperature of the electrolyte using the temperature sensor 121; and calibrates the electric conductivity sensor 120 based on the difference between the measured temperature and a known temperature of the electrolyte by referring to the correlation between temperature and electric conductivity.

More specifically, first, in the therapeutic fluid preparation device 60 in a preparation stage before starting the blood treatment, an electrolyte having a known concentration (i.e., an electrolyte whose temperature and electric conductivity are known) in the electrolyte supply source 130 is supplied to the solution mixture preparation circuit 81 by the electrolyte supply means 72, and allowed to flow through the solution mixture preparation circuit 81. Then, the temperature of the electrolyte flowing through the solution mixture preparation circuit 81 is measured by the temperature sensor 121 of the concentration measuring device 71. The result of the temperature measurement is output to the calibration means 73. The calibration means 73 determines the difference between the measured temperature of the electrolyte measured by the temperature sensor 121 and the known temperature of the same electrolyte, and considers the obtained difference as being an electric conductivity factor, so that the calibration means 73 calibrates the electric conductivity sensor 120 based on the difference by referring to the correlation between temperature and electric conductivity.

To be more specific, as already stated, the above calibration is based on the premise that electric conductivity and temperature vary with a certain correlation therebetween (electric conductivity variation $\Delta C = \beta$ x temperature variation $\Delta T$ (electric conductivity varies by $\beta$ mS/cm when temperature varies by 1°C)). Then, the difference $\Delta M$ between the temperature measured by the temperature sensor 121 and the known temperature of the electrolyte is considered to be entirely due to the electric conductivity sensor, i.e., the difference $\Delta M$ is considered to be an error of the electric conductivity sensor 120 and is therefore converted into a calibration amount N for the electric conductivity sensor 120. Such calibration amount N for the electric conductivity sensor 120 can be determined from the above-mentioned correlation between the temperature and electric conductivity and the difference $\Delta M$. More specifically, since the electric conductivity varies by $\beta$ mS/cm if the temperature varies by 1°C, the calibration amount N for the electric conductivity sensor 120 can be determined by the following expression (2):

$$N = \Delta M \times \beta \quad \ldots\ldots\ldots\ldots (2).$$

For example, in the case where a 150 mmol/L sodium ion solution is used as an electrolyte and the electric conductivity thereof varies by 2 mS/cm when the temperature varies by 1°C (i.e., $\beta = 2$), and if the known temperature and the measured temperature are 25°C and 24°C, respectively, the difference $\Delta M$ between the known temperature and the measured temperature is 1°C, and the calibration amount N for the electric conductivity sensor 120 is calculated as: 1 x 2 = 2 mS/cm according to the above expression (2). Accordingly, the electric conductivity sensor 120 is calibrated by the obtained calibration amount N (2 mS/cm). As a result, the concentration measuring device 71, which measures concentration by way of the electric conductivity sensor 120 and the temperature sensor 121, can be calibrated.

According to the present embodiment: the electrolyte supply means 72 supplies an electrolyte having a known concentration to the mixing circuit 70 and allows it to flow through the mixing circuit 70; the temperature sensor 121 measures the temperature of such electrolyte having the known concentration; and the electric conductivity sensor 120 is calibrated based on the difference between the measured temperature and a known temperature in accordance with the correlation between the temperature and electric conductivity. As a result, the present embodiment enables the calibration of the concentration measuring device 71 in the therapeutic fluid preparation device 60 without increasing the burden to healthcare workers during the preparation stage for treatment and also without the need to add further functions to the sensors, thereby improving the measurement accuracy of the concentration measuring device 71.

The concentration measuring device 71 is calibrated through the calibration of the temperature sensor 121 in the first embodiment, while the concentration measuring device 71 is calibrated through the calibration of the electric conductivity sensor 120 in the second embodiment, but both the calibration through the temperature sensor 121 and the calibration through the electric conductivity sensor 120 may be performed. In that case, calibration through the temperature sensor 121 may be performed first and calibration through the electric conductivity sensor 120 may be performed later, and vice versa. Alternatively, calibration through the temperature sensor 121 and calibration through the electric conductivity sensor 120 may be performed at the same time.

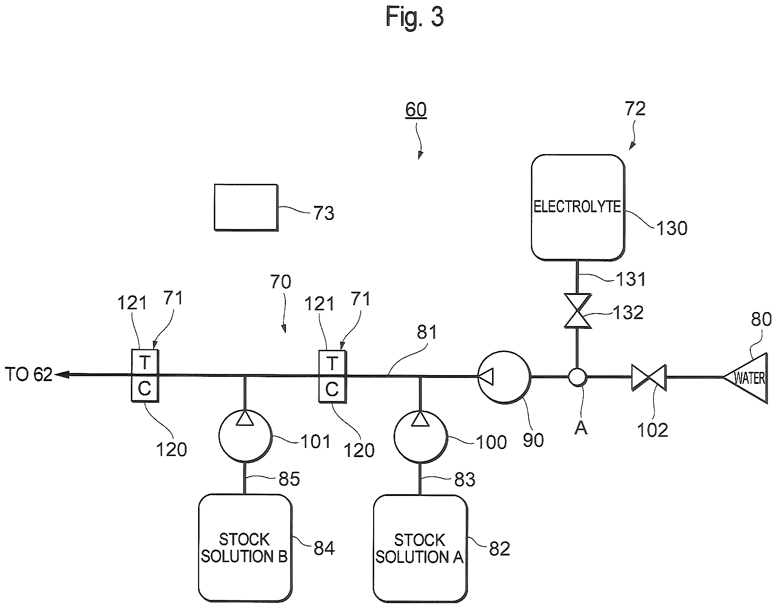

Although the therapeutic fluid preparation device 60 in the above-described first and second embodiments has a single concentration measuring device 71, the therapeutic fluid preparation device 60 may have two or more concentration measuring devices 71. In that case, all of the concentration measuring devices 71 may be configured so as to be calibrated. For example, as shown in Fig. 3, a concentration measuring device 71 may be provided between the connection point in the solution mixture preparation circuit 81 with the stock solution-A supply circuit 83 and the connection point with the stock solution-B supply circuit 85. This configuration enables the measuring of the concentration of the solution mixture after the mixing of stock solution-A and the measuring of the concentration of the solution mixture after the further mixing of stock solution-B, and therefore, the mixing ratio between stock solutions-A and B can be measured. In such configuration, the burden to healthcare workers can be further reduced by calibrating the two concentration measuring devices 71 in the same manner as in the above-described embodiments.

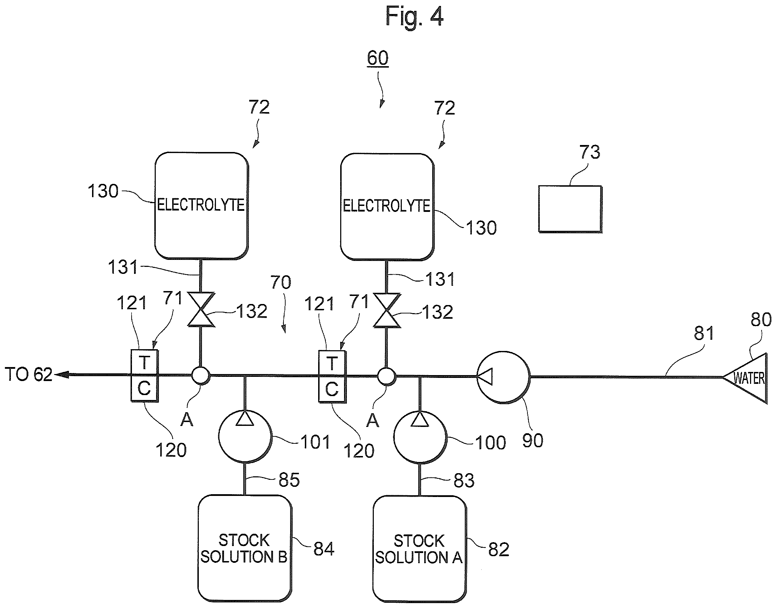

In the case where two or more concentration measuring devices 71 are provided as described above, the electrolyte supply means 72 may be provided for each of the concentration measuring devices 71, as illustrated in Fig. 4. In that case, for example, the electrolyte supply circuit 131 of a first electrolyte supply means 72 may be connected to the solution mixture preparation circuit 81 at a point between a first concentration measuring device 71 and the connection point with the stock solution-A supply circuit 83; whereas, the electrolyte supply circuit 131 of a second electrolyte supply means 72 may be connected to the solution mixture preparation circuit 81 at a point between a second concentration measuring device 71 and the connection point with the stock solution-B supply circuit 85. Such first and second electrolyte supply means 72 may be configured so as to send the electrolyte in the corresponding electrolyte supply sources 130 using a syringe so as to allow the electrolyte to flow through the solution mixture preparation circuit 81. Further, the first and second electrolyte supply means 72 may be configured so as to supply the electrolyte to the solution mixture preparation circuit 81 in turns, and, for example, the concentration of the electrolyte supplied by the first electrolyte supply means 72 may be measured by the first concentration measuring device 71 (i.e., the temperature or electric conductivity sensor) so as to calibrate the first concentration measuring device 71 in the same manner as in the above-described embodiment, and, after that, the concentration of the electrolyte supplied by the second electrolyte supply means 72 may be measured by the second concentration measuring device 71 (i.e., the temperature or electric conductivity sensor) so as to calibrate the second concentration measuring device 71. It should be noted here that the amount of electrolyte used can be reduced when the electrolyte supply means 72 is a shorter distance from the target sensor to be calibrated and, furthermore, the accuracy of the calibration is also improved in such case. Such benefit is likely to be obtained by providing two electrolyte supply means 72.

Third embodiment, not forming part of the invention

A third embodiment will be described next, in which the concentration of the solution mixture, which is to be measured by the concentration measuring device 71, is calculated based on the fact that the ratio of the concentration of a solution mixture and the concentration of an electrolyte (ion solution) and the ratio of the electric conductivity of the solution mixture and the electric conductivity of the electrolyte are approximately constant within certain temperature and concentration ranges, as represented by the following expression (3):

$$\text{(Electrolyte electric conductivity)} / \text{(Solution mixture electric conductivity)} = \alpha * \text{(Electrolyte concentration)} / \text{(Solution mixture concentration)} \ \ldots\ldots\ (3)$$

where $\alpha$ is a constant.

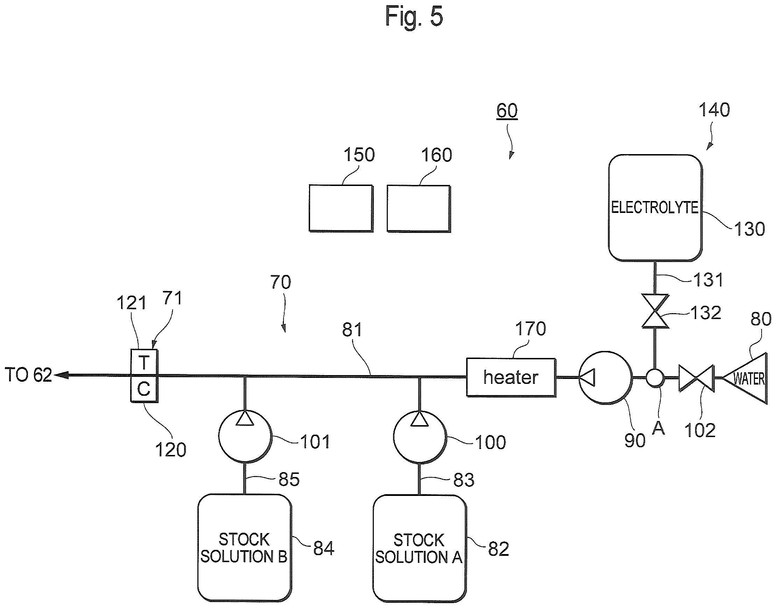

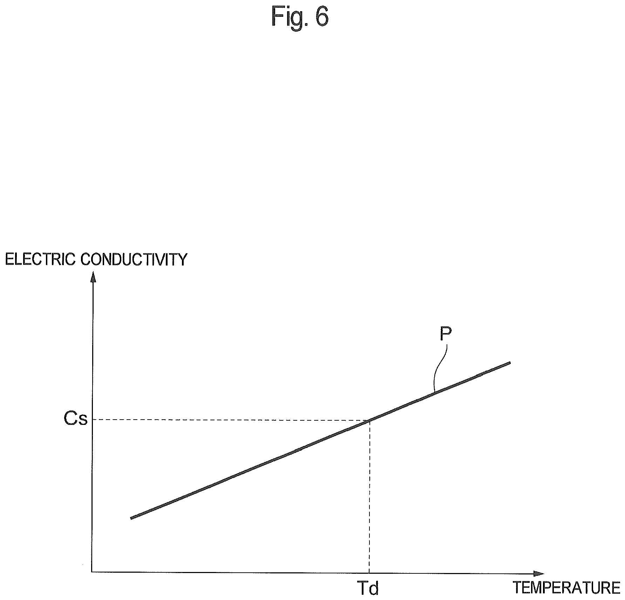

In this embodiment, as illustrated in Fig. 5, the therapeutic fluid preparation device 60 includes, for example, a mixing circuit 70 and a concentration measuring device 71 which are similar to those in the above-described embodiments, and the therapeutic fluid preparation device 60 also includes: an electrolyte supply means 140 that allows an electrolyte, such as a sodium ion solution, having a known concentration to flow through the mixing circuit 70, with the temperature of the electrolyte being changed; a calibration curve generating means 150 that generates a calibration curve P (shown in Fig. 6) for electric conductivity and temperature by measuring the electric conductivity and temperature of the electrolyte flowing through the mixing circuit 70 with varying temperatures using the electric conductivity sensor 120 and the temperature sensor 121; a concentration calculating means 160 that measures the electric conductivity and temperature of a solution mixture in the mixing circuit 70 using the electric conductivity sensor 120 and the temperature sensor 121, that determines, on the calibration curve P, the electric conductivity of the electrolyte at the same temperature as the measured temperature of the solution mixture, and that calculates the concentration of the solution mixture based on the specified relational expression (3).

The electrolyte supply means 140 includes similar components to those of the electrolyte supply means 72 in the above-described embodiments, such as an electrolyte supply source 130, an electrolyte supply circuit 131, an opening/closing means 132 and a liquid feeding means 90, and the electrolyte supply means 140 further includes, for example, a heater 170 serving as a heating device for changing the temperature of the electrolyte. Such heater 170 is disposed in, for example, the solution mixture preparation circuit 81 so that the temperature of the electrolyte flowing through the solution mixture preparation circuit 81 can be changed.

The calibration curve generating means 150 is, for example, a computer, which, upon the execution of programs stored on the memory thereof: causes the electrolyte supply means 140 to supply an electrolyte having a known concentration so as to flow through the mixing circuit 70 with varying temperatures; measures the electric conductivity and temperature of the supplied electrolyte using the electric conductivity sensor 120 and the temperature sensor 121; and generates a calibration curve P for electric conductivity and temperature based on the results of the measurement.

The concentration calculating means 160 is, for example, a computer, which, upon the execution of programs stored on the memory thereof: refers to the electric conductivity and temperature of the solution mixture in the mixing circuit 70 measured by the electric conductivity sensor 120 and temperature sensor 121, so as to obtain, on the calibration curve P generated by the calibration curve generating means 150, the electric conductivity of the electrolyte at the same temperature as the measured temperature of the solution mixture; and calculates the concentration of the solution mixture based on the relational expression (3).

The above calculation of the concentration of the solution mixture is performed in the therapeutic fluid preparation device 60 according to the following process. First, in a preparation stage before starting continuous blood treatment or the like, a calibration curve P is generated from the electric conductivity and temperature measured by the electric conductivity sensor 120 and the temperature sensor 121 of the concentration measuring device 71. In this operation, under the command from the calibration curve generating means 150, the electrolyte supply means 140 supplies from the electrolyte supply source 130 an electrolyte having a known concentration (Fs) to the solution mixture preparation circuit 81, and the temperature thereof is gradually increased by the heater 170. Then, the electric conductivity and temperature of the electrolyte are continuously measured with the electric conductivity sensor 120 and the temperature sensor 121, thereby obtaining measurements varying with time. After that, a calibration curve P for electric conductivity and temperature as shown in Fig. 6 is generated from the obtained electric conductivity and temperature measurements.

For example, in order to actually measure the concentration of the solution mixture in the therapeutic fluid preparation device 60 at the time of treatment, the electric conductivity Cd and temperature Td of the solution mixture are first measured by the electric conductivity sensor 120 and the temperature sensor 121 of the concentration measuring device 71. Then, as shown in Fig. 6, the electric conductivity Cs of the electrolyte at the temperature Td is determined from the calibration curve P. The known concentration Fs of the electrolyte, the electric conductivity Cs of the electrolyte, and the electric conductivity Cd of the solution mixture are substituted into the relational expression (3), and the expression is then modified so that the concentration Fd of the solution mixture is determined by the following expression (4):

$$Fd = \alpha * Fs * Cd/Cs \quad \ldots\ldots \ (4)$$

Here, the value of $\alpha$ is determined in advance through experimentation, etc.

According to the present embodiment, the electrolyte supply means 140 supplies an electrolyte having a known concentration to flow with varying temperatures, and the electric conductivity and temperature of such electrolyte are measured using the electric conductivity sensor 120 and the temperature sensor 121 so as to accordingly generate a calibration curve P for the electric conductivity and the temperature. The generated calibration curve P may include an error due to the electric conductivity sensor 120 or the temperature sensor 121. Then, at the time of measuring the concentration of a solution mixture for continuous treatment, the electric conductivity sensor 120 and the temperature sensor 121 are used to measure the electric conductivity Cd and the temperature Td of the solution mixture, and the electric conductivity Cs of the electrolyte at the temperature Td is then determined from the calibration curve P. After that, the concentration Fd of the solution mixture can be determined using the relational expression (3), based on the electric conductivity Cs of the electrolyte, the known concentration Fs of the electrolyte, and the measured electric conductivity Cd of the solution mixture. As a result, measurement errors due to the concentration measuring device 71 can be reduced and the measurement accuracy of the concentration measuring device 71 in the therapeutic fluid preparation device 60 can be improved without increasing the burden to healthcare workers at a preparation stage for treatment, such as continuous hemofiltration treatment, and without the need to add further functions to the sensors.

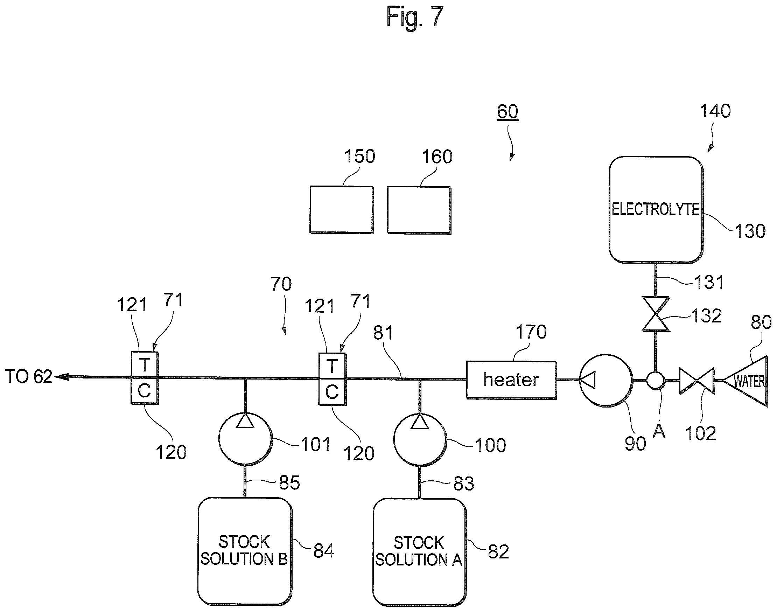

In this third embodiment as well, two concentration measuring devices 71 may be provided as shown in Fig. 7. In that case, a calibration curve P for the electric conductivity and the temperature may be generated for each of the concentration measuring devices 71, so that the concentration of the solution mixture is calculated by the concentration measuring devices 71 using the respective calibration curves P. Alternatively, a calibration curve P may be generated for either of the two concentration measuring devices 71, so that the concentration of the solution mixture is calculated by the two concentration measuring devices 71 using the one calibration curve P. In order to further improve the calculation accuracy, it is preferable to use an electrolyte containing the same kind of ions as those in the solution mixture.

Preferred embodiments of the present invention have been described above by referring to the attached drawings, but the present invention is not limited to the described examples. A person skilled in the art could obviously think of various alterations and modification without departing from the idea specified in the scope of the claims and it should be appreciated that such alterations and modifications would obviously be encompassed within the technical scope of the present invention.

For example, the configuration of the mixing circuit 70 in the therapeutic fluid preparation device 60 is not limited to that in the above-described embodiments. For example, although the stock solution-A supply circuit 83 is connected to the solution mixture preparation circuit 81 at a point upstream of the stock solution-B supply circuit 85, it may be connected downstream of the stock solution-B supply circuit 85. Although the above embodiments describe an example in which two kinds of stock solutions, namely, stock solutions A and B, are mixed, the present invention can also be applied to examples in which three or more kinds of stock solutions are mixed. Furthermore, the therapeutic fluid supply device 11, to which the therapeutic fluid preparation device 60 is connected, may have a different configuration from that described above, and the blood processing device 10 may also have a different configuration from that described above. Moreover, the therapeutic fluid preparation device 60 may also be used for supplying therapeutic fluid to a blood processing device for performing treatment other than continuous treatment.

Industrial Applicability

The present invention is useful for improving the measurement accuracy of a concentration measuring device of a therapeutic fluid preparation device, without increasing the burden to healthcare workers during a preparation stage for treatment, such as continuous treatment, and without the need to add further functions to a sensor.

Reference Signs List

| 1 | Blood processing system |
| 10 | Blood processing device |
| 11 | Therapeutic fluid supply device |
| 60 | Therapeutic fluid preparation device |
| 70 | Mixing circuit |
| 71 | Concentration measuring device |
| 72 | Electrolyte supply means |
| 73 | Calibration means |
| 83 | Stock solution-A supply circuit |
| 85 | Stock solution-B supply circuit |
| 150 | Calibration curve generating means |
| 160 | Concentration calculating means |

**Claims**

**1.**    A therapeutic fluid preparation device (60) for preparing therapeutic fluid having a predetermined concentration by mixing a plurality of stock solutions, the device (60) comprising:

a mixing circuit (70) configured to mix the plurality of stock solutions;
a concentration measuring device (71) having an electric conductivity sensor configured to measure the electric conductivity of the solution mixture in the mixing circuit (70) and a temperature sensor (121) configured to measure the temperature of the solution mixture, the concentration measuring device (71) being configured to measure the concentration of the solution mixture in accordance with the measured electric conductivity by the electric conductivity sensor (120) and the measured temperature by the temperature sensor (121);
electrolyte supply means (72) configured to supply an electrolyte whose temperature and electric conductivity is known through the mixing circuit; and
calibration means (73) configured to direct the electrolyte supply means (72) to supply the electrolyte whose temperature and electric conductivity is known through the mixing circuit (70), that is configured to measure the electric conductivity of the supplied electrolyte by means of the electric conductivity sensor (120), and that is configured to calibrate the temperature sensor (121) based on the difference between the measured electric conductivity and the known electric conductivity of the electrolyte by referring to a correlation between temperature and electric conductivity.

2. The therapeutic fluid preparation device (60) according to claim 1, wherein at least one of the electric conductivity sensor ($12_0$) and the temperature sensor (121) is disposable.

3. The therapeutic fluid preparation device (60) according to claim 1 or 2, wherein the electrolyte is a priming solution.

4. A blood processing system (1) for performing continuous blood treatment, comprising a therapeutic fluid preparation device (60) according to any of claims 1 to 3.


**Patentansprüche**

1. Herstellungsvorrichtung (60) für eine therapeutische Flüssigkeit zur Herstellung einer therapeutischen Flüssigkeit mit einer vorbestimmten Konzentration durch Mischen einer Vielzahl von Stammlösungen, wobei die Vorrichtung (60) umfasst:

eine Mischleitung (70), die so konfiguriert ist, dass sie die Vielzahl von Stammlösungen mischen kann;
eine Konzentrationsmessvorrichtung (71) mit einem Sensor für elektrische Leitfähigkeit, der so konfiguriert ist, dass er die elektrische Leitfähigkeit des Lösungsgemischs in der Mischleitung (70) messen kann, und einem Temperatursensor (121), der so konfiguriert ist, dass er die Temperatur des Lösungsgemischs messen kann, wobei die Konzentrationsmessvorrichtung (71) so konfiguriert ist, dass sie die Konzentration des Lösungsgemischs im Einklang mit der vom Sensor für elektrische Leitfähigkeit (120) gemessenen elektrischen Leitfähigkeit und der vom Temperatursensor (121) gemessenen Temperatur messen kann;
eine Elektrolyt-Zufuhreinrichtung (72), die so konfiguriert ist, dass sie einen Elektrolyten, dessen Temperatur und elektrische Leitfähigkeit bekannt sind, durch die Mischleitung zuführt; und
eine Kalibrierungseinrichtung (73), die so konfiguriert ist, dass sie die Elektrolyt-Zufuhreinrichtung (72) so steuert, dass diese den Elektrolyten, dessen Temperatur und elektrische Leitfähigkeit bekannt sind, durch die Mischleitung (70) zuführt, die so konfiguriert ist, dass sie die elektrische Leitfähigkeit des zugeführten Elektrolyten mittels des Sensors für elektrische Leitfähigkeit (120) misst, und die so konfiguriert ist, dass sie den Temperatursensor (121) auf der Basis der Differenz zwischen der gemessenen elektrischen Leitfähigkeit und der bekannten elektrischen Leitfähigkeit des Elektrolyten kalibriert, indem sie sich auf eine Korrelation zwischen Temperatur und elektrischer Leitfähigkeit bezieht.

2. Herstellungsvorrichtung (60) für eine therapeutische Flüssigkeit gemäß Anspruch 1, wobei der Sensor für elektrische Leitfähigkeit (120) und/oder der Temperatursensor (121) Einweggeräte sind.

3. Herstellungsvorrichtung (60) für eine therapeutische Flüssigkeit gemäß Anspruch 1 oder 2, wobei der Elektrolyt eine vorbereitende Lösung ist.

4. Blutverarbeitungssystem (1) zur Durchführung einer kontinuierlichen Blutbehandlung, umfassend eine Herstellungsvorrichtung (60) für eine therapeutische Flüssigkeit gemäß einem der Ansprüche 1 bis 3.

**Revendications**

1. Dispositif de préparation de fluide thérapeutique (60) pour préparer un fluide thérapeutique ayant une concentration prédéterminée en mélangeant une pluralité de solutions mères, le dispositif (60) comprenant :

   un circuit de mélange (70) configuré pour mélanger la pluralité de solutions mères ;
   un dispositif de mesure de concentration (71) ayant un capteur de conductivité électrique configuré pour mesurer la conductivité électrique du mélange de solution dans le circuit de mélange (70) et un capteur de température (121) configuré pour mesurer la température du mélange de solution, le dispositif de mesure de concentration (71) étant configuré pour mesurer la concentration du mélange de solution conforme à la conductivité électrique mesurée par le capteur de conductivité électrique (120) et de la température mesurée par le capteur de température (121) ;
   des moyens d'alimentation en électrolyte (72) configurés pour alimenter un électrolyte dont la température et la conductivité électrique sont connues par l'intermédiaire du circuit de mélange ; et
   des moyens d'étalonnage (73) configurés pour diriger les moyens d'alimentation en électrolyte (72) afin d'alimenter l'électrolyte dont la température et la conductivité électrique sont connues par l'intermédiaire du circuit de mélange (70), qui sont configurés pour mesurer la conductivité électrique de l'électrolyte alimenté au moyen du capteur de conductivité électrique (120), et qui sont configurés pour étalonner le capteur de température (121) sur la base de la différence entre la conductivité électrique mesurée et la conductivité électrique connue de l'électrolyte en se référant à une corrélation entre la température et la conductivité électrique.

2. Dispositif de préparation de fluide thérapeutique (60) selon la revendication 1, dans lequel au moins l'un parmi le capteur de conductivité électrique (120) et le capteur de température (121) est jetable.

3. Dispositif de préparation de fluide thérapeutique (60) selon la revendication 1 ou 2, dans lequel l'électrolyte est une solution d'amorçage.

4. Système de traitement de sang (1) pour effectuer un traitement sanguin en continu, comprenant un dispositif de préparation de fluide thérapeutique (60) selon l'une quelconque des revendications 1 à 3.

Fig. 1

EP 3 254 712 B1

Fig. 2

Fig. 3

EP 3 254 712 B1

## Fig. 4

60

72

72

73

130

ELECTROLYTE

ELECTROLYTE

130

131

131

70

121  71  132

121  71  132

120

T
C

T
C

TO 62

A

A

120

120

101

100

90

81

80

WATER

85

83

STOCK
SOLUTION B

84

STOCK
SOLUTION A

82

Fig. 5

Fig. 6

Fig. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9411093 A
- US 20070255527 A

- JP 4458346 B